# EUROPEAN PATENT APPLICATION

(11) **EP 2 027 848 A2**
(43) Date of publication of application: **25.02.2009**
(21) Application number: 08014668.1
(22) Date of filing: 19.08.2008
(51) Int. Cl.: A61K 8/63, A61Q 19/10

(54) **Liquid skin cleanser with multiple signals of adequate wash duration with adequate mechanical force**

(30) Priority: 24.08.2007 US 966091 P; 12.02.2008 US 69640
(71) Applicant: The Dial Corporation, Scottsdale, AZ 85260-1619 (US)
(72) Inventor: Schmit, Catherine, Glendale AZ 85308 (US); Dalton, James J., Scottsdale AZ 85254 (US); Goldfarb, Heidi B., Scottsdale AZ 85255 (US)
(74) Representative: Augustin-Castro, Barbara

(57) **Abstract**

A personal cleansing composition includes a carrier, and a first plurality of beads entrained in the carrier. The beads impart, to a user applying the cleansing composition with hand rubbing force, perceivable tactility for a limited predetermined duration that corresponds to a minimum adequate cleansing time period. In accordance with another embodiment of the present invention, the personal cleansing composition further includes a second plurality of beads entrained in the carrier. The second plurality of beads imparts to the user a perceivable change in color for the cleansing composition for a second limited predetermined duration that is substantially equal to the duration that the tactility imparted by the first plurality of beads is perceivable to the user.

## Description

The present invention is directed to a personal cleansing composition. More particularly, the present invention is directed to a cleansing composition that is adapted to provide signals to encourage a person using the composition to wash until proper cleansing has occurred.

Cleansing compositions such as body washing agents typically include a carrier system that may have conventional perceivable sensorial attributes. Efforts are continually made to make the sensorial attributes of cleansing compositions appealing to users. Fragrances, colorants, temperature change imparting agents (both endothermic and exothermic), and tactility modifying agents are just a few examples of components that provide sensorial attributes to cleansing compositions to induce consumers to purchase and use particular compositions. However, such components are not typically adapted with a view toward improving cleaning efficacy. In other words, cleansing components and agents that provide sensorial attributes to compositions are typically considered separately when developing and implementing cleansing formulations. Consequently, conventional perceived sensorial attributes of a composition are not usually coupled or coordinated with particular hand washing events in a manner that increases the probability that a user will comply with proper body cleansing requirements.

A few recent developments reflect efforts by some to produce cleansing compositions that tie the concepts of sensorial attributes with adequate cleansing. For example, WO 2004/052307 discloses hand and hair cleaning compositions that include a carrier, and a plurality of capsules within the carrier. The capsules contain and, while washing using the cleaning composition, release a material that provides a sensorial stimulus. The capsules are adapted to release the material at a particular time that is coincident with a discrete event such as lapse of an adequate hand cleaning time. Some materials that may be contained in the capsules are colorants or fragrances that, when seen or smelled by the user, indicate that the discrete event has occurred. Other materials that may be contained in the capsules are pH modifiers that, when released, change the alkalinity or acidity of the carrier, and consequently change the perceived color of the overall composition. Yet another material that may be contained in the capsules is an acidic component that, when released, reacts with an alkaline carrier and produces bubbles and an audible sound, along with a tactile sensation corresponding to the bubble formation.

One limitation of the prior art cleaning solutions is that users are likely to discontinue washing as soon as any sensorial indicia are produced. This is particularly a problem when the sensorial indicia include color changes produced by release of colorants from a capsule or other bead included in a carrier solution. If a consumer discontinues scrubbing as soon as a color change is perceived, then rinsing the cleansing solution will cause several intact beads to fall into a sink or other surface, and collapsing of the beads will produce smears or other messy residue. Accordingly, there is a need for personal cleansing compositions that provide sensorial indicia that indicate adequate washing to a user. There is also a need for such compositions that ensure the usage of substantially all of the sensorial indicia before a user rinses the cleansing composition.

This summary of the invention section is intended to introduce the reader to aspects of the invention. Particular aspects of the invention are pointed out in other sections herein below, and the invention is set forth in the appended claims which alone demarcate its scope.

In accordance with an exemplary embodiment of the present invention, a personal cleansing composition is provided, which includes a carrier, and a first plurality of beads entrained in the carrier. The beads impart, to a user applying the cleansing composition with hand rubbing force, perceivable tactility for a limited predetermined duration that corresponds to a minimum adequate cleansing time period.

In accordance with another exemplary embodiment of the present invention, the personal cleansing composition further includes a second plurality of beads entrained in the carrier. The second plurality of beads imparts to the user a perceivable change in color for the cleansing composition for a second limited predetermined duration that is substantially equal to the duration that the tactility imparted by the first plurality of beads is perceivable to the user.

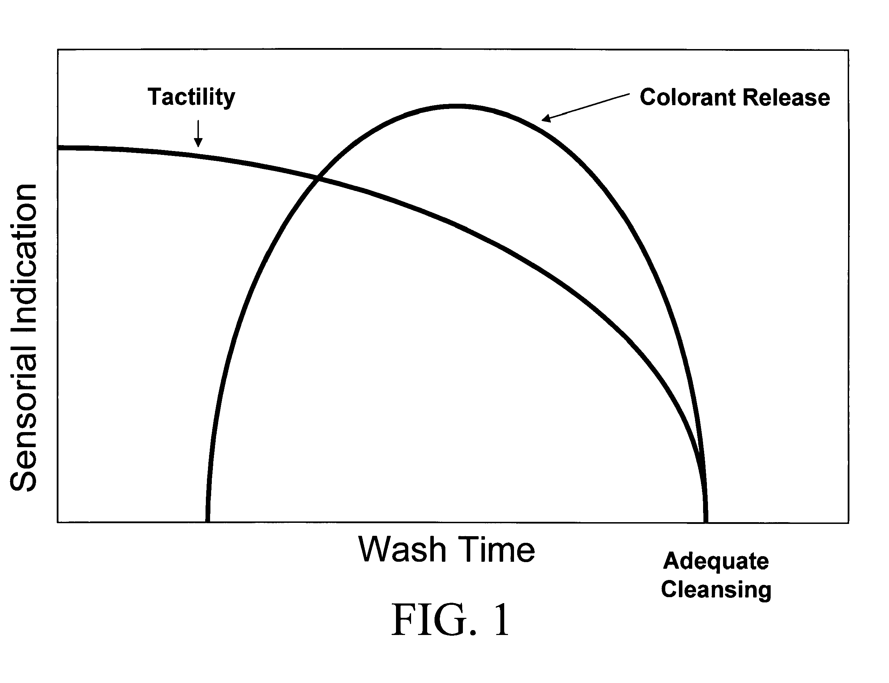

FIG. 1 is a graph depicting sensorial intensity provided to a user applying a hand rubbing force to beads included in a cleansing composition during a washing event, and correlates the sensorial intensity with the duration of the washing event.

The detailed description of various exemplary embodiments of the invention herein makes reference to exemplary compositions and methods of process for producing such compositions. While these exemplary embodiments are described in sufficient detail to enable those skilled in the art to practice the invention, it should be understood that other embodiments may be realized, and that logical and processing changes may be made without departing from the spirit and scope of the invention. Thus, the detailed description herein is presented for purposes of illustration only and not of limitation.

According to one embodiment, a cleansing composition includes a carrier with a first plurality of beads entrained therein. The beads imparting perceived tactility to a user applying the cleansing composition with a hand rubbing force. The tactility is perceivable by the user for a limited predetermined duration that corresponds to a minimum adequate cleansing time period. The beads are preferably formed as a uniform composition that is adapted to disintegrate after adequate cleansing occurs to the extent that the beads no longer impart perceivable tactility to the user.

The bead-containing cleansing compositions of the present invention are primarily contemplated for use as personal cleansing agents. However, other bead-containing cleansing compositions that impart perceived tactility to the user and that employ the principles of the present invention may be adapted for functions other than personal cleansing. When used for personal cleansing, the compositions may be useful as agents for washing human skin, hair and/or nails. The cleansing compositions may be included in various formulations including liquids, creams, gels, foams, lotions, mousses, and powders, to name a few examples.

The beads are included in a suitable carrier, which includes aqueous and/or water-miscible solutions in which the beads of the present inventions can be maintained without substantial degradation during non-use of the cleaning composition. According to a preferred embodiment, the carrier is aqueous and includes at least about 50% water by weight. Other alcohol-based carriers that may also be useful in combination with water include, as just a few examples, various aliphatic straight and branched alcohols, glycols, and alcohols of polyethers. Especially preferred are ethanol and 1,2-propylene glycol as well as mixtures of ethanol and 1,2-propylene glycol and mixtures of ethanol, 1,2-propylene glycol and water.

The compositions in accordance with the invention also include at least one surfactant for the purpose of forming homogenous solutions or for dispersion or suspension of components for the purpose of creating a heterogenous system such as an emulsion or suspension. The plurality of surfactants may include anionic, cationic, nonionic, and/or amphoteric surfactants. Some exemplary anionic surfactants include sodium laureth sulfate, sodium toluene sulfonate, and sodium naphthalene sulfonate. Some exemplary cationic surfactants include alkyl, aryl, and alkyl aryl amines. Some exemplary nonionic surfactants include ethoxylates such as alkyl ethoxylates, aryl ethoxylates, alkyl aryl ethoxylates, fatty acid ethoxylates, and esters of such ethoxylates with other compounds. Amphoteric surfactants (also called zwitterionic surfactants) are those having a positive and negative charge within the same molecule and therefore are a blend of cationic and anionic. A common example is the class of betaine surfactants.

The surfactant-containing cleansing composition of the invention comprises - based on its weight - 5 to 30% by weight, preferably 7 to 20% by weight and in particular 8 to 14% by weight, of surfactants. These are selected from the group of anionic, amphoteric, zwitterionic and nonionic surfactants, and from mixtures of these surfactant classes.

Suitable anionic surfactants in the preparations according to the invention are all anionic surface-active substances suitable for use on the human body. These are characterized by a water-solubilizing, anionic group such as, for example, a carboxylate group, sulfate group, sulfonate group or phosphate group, and a lipophilic alkyl group having about 8 to 30 carbon atoms. In addition, glycol or polyglycol ether groups, ester groups, ether groups and amide groups, and hydroxyl groups may be present in the molecule. Examples of suitable anionic surfactants are, in each case in the form of the sodium, potassium and ammonium salts, and the mono-, di- and trialkanolammonium salts having 2 to 4 carbon atoms in the alkanol group,
- linear and branched fatty acids having 8 to 30 carbon atoms (soaps),
- ether carboxylic acids of the formula
   R-O-(CH₂-CH₂O)ₓ-CH₂-COOH, in which R is a linear alkyl group having 8 to 30 carbon atoms and x is 0 or 1 to 16,
- acyl sarcosides having 8 to 24 carbon atoms in the acyl group,
- acyl taurides having 8 to 24 carbon atoms in the acyl group,
- acyl isethionates having 8 to 24 carbon atoms in the acyl group,
- sulfosuccinic mono- and dialkyl esters having 8 to 24 carbon atoms in the alkyl group and sulfosuccinic acid monoalkylpolyoxyethyl esters having 8 to 24 carbon atoms in the alkyl group and 1 to 6 oxyethyl groups,
- linear alkanesulfonates having 8 to 24 carbon atoms,
- linear alpha-olefinsulfonates having 8 to 24 carbon atoms,
- alpha-sulfo fatty acid methyl esters of fatty acids having 8 to 30 carbon atoms,
- alkyl sulfates and alkyl polyglycol ether sulfates of the formula R-O(CH₂-CH₂O)ₓ-OSO₃H, in which R is a preferably linear alkyl group having 8 to 30 carbon atoms and x is 0 or 1 to 12,
- mixtures of surface-active hydroxysulfonates as in DE-A-37 25 030,
- sulfated hydroxyalkyl polyethylene and/or hydroxyalkylene propylene glycol ethers as in DE-A-37 23 354,
- sulfonates of unsaturated fatty acids having 8 to 24 carbon atoms and 1 to 6 double bonds as in DE-A-39 26 344,
- esters of tartaric acid and citric acid with alcohols, which are addition products of about 2-15 molecules of ethylene oxide and/or propylene oxide onto fatty alcohols having 8 to 22 carbon atoms,
- alkyl and/or alkenyl ether phosphates of the formula (II) 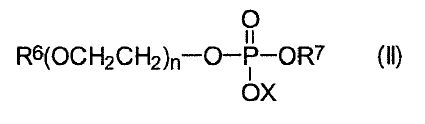 in which R⁶ is preferably an aliphatic hydrocarbon radical having 8 to 30 carbon atoms, R⁷ is hydrogen, a radical (CH₂CH₂O)ₙR⁶ or X, n is numbers from 1 to 10 and X is hydrogen, an alkali metal or alkaline earth metal or NR⁸R⁹R¹⁰R¹¹, where R⁸ to R¹¹, independently of one another, are hydrogen or a C₁ to C₄ hydrocarbon radical,
- sulfated fatty acid alkylene glycol esters of the formula (III)

   R¹²CO(AlkO)ₙSO₃M (III)

   in which R¹²CO- is a linear or branched, aliphatic, saturated and/or unsaturated acyl radical having 6 to 22 carbon atoms, Alk is CH₂CH₂, CHCH₃CH₂ and/or CH₂CHCH₃, n is numbers from 0.5 to 5 and M is a cation, as are described in DE-A 197 36 906.5,
- monoglyceride sulfates and monoglyceride ether sulfates of the formula (IV), as have been described, for example, in EP-B1 0 561 825, EP-B1 0 561 999, DE-A1 42 04 700 or by A.K. Biswas et al. in J. Am. Oil. Chem. Soc. 37, 171 (1960) and F.U. Ahmed in J. Am. Oil. Chem. Soc. 67, 8 (1990), 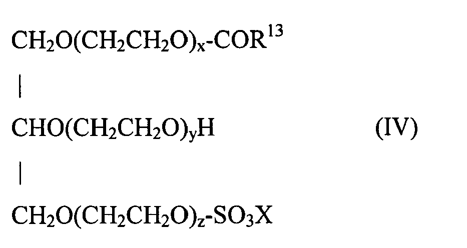
in which R¹³CO is a linear or branched acyl radical having 6 to 22 carbon atoms, x, y and z are in total 0 or numbers from 1 to 30, preferably 2 to 10, and X is an alkali metal or alkaline earth metal. Typical examples of monoglyceride (ether) sulfates suitable for the purposes of the invention are the reaction products of lauric acid monoglyceride, coconut fatty acid monoglyceride, palmitic acid monoglyceride, stearic acid monoglyceride, oleic acid monoglyceride and tallow fatty acid monoglyceride, and ethylene oxide adducts thereof with sulfur trioxide or chlorosulfonic acid in the form of their sodium salts. Preference is given to using monoglyceride sulfates of the formula (VIII) in which R¹³CO is a linear acyl radical having 8 to 18 carbon atoms.

Preferred anionic surfactants are alkyl sulfates, alkyl polyglycol ether sulfates and ether carboxylic acid salts having 10 to 18 carbon atoms in the alkyl group and up to 12 glycol ether groups in the molecule and sulfosuccinic acid mono- and dialkyl esters having 8 to 18 carbon atoms in the alkyl group and sulfosuccinic acid monoalkylpolyoxyethylene esters having 8 to 18 carbon atoms in the alkyl group and 1 to 6 ethylene oxide groups.
Particularly preferred anionic surfactants are the alkali metal or ammonium salts of lauryl ether sulfate with a degree of ethoxylation of from 2 to 4 EO.

Zwitterionic surfactants is the term used to refer to those surface-active compounds which carry at least one quaternary ammonium group and at least one -COO⁽⁻⁾ or - SO₃⁽⁻⁾ group in the molecule. Particularly suitable zwitterionic surfactants are the so-called betaines, such as the N-alkyl-N,N-dimethylammonium glycinates, for example cocoalkyl-dimethylammonium glycinate, N-acylaminopropyl-N,N-dimethylammonium glycinates, for example cocoacylaminopropyldimethylammonium glycinate, and 2-alkyl-3-carboxymethyl-3-hydroxyethylimidazolines having in each case 8 to 18 carbon atoms in the alkyl or acyl group, and cocoacylaminoethyl hydroxyethylcarboxymethyl glycinate. A preferred zwitterionic surfactant is the fatty acid amide derivative known under the INCI name Cocamidopropyl Betaine.

Ampholytic surfactants are understood as meaning those surface-active compounds which, apart from a C₈-C₂₄-alkyl or -acyl group in the molecule, comprise at least one free amino group and at least one -COOH or -SO₃H group and are capable of forming internal salts. Examples of suitable ampholytic surfactants are N-alkylglycines, N-alkylpropionic acids, N-alkylaminobutyric acids, N-alkyliminodipropionic acids, N-hydroxyethyl-N-alkylamidopropylglycines, N-alkyltaurines, N-alkylsarcosines, 2-alkylaminopropionic acids and alkylaminoacetic acids having in each case about 8 to 24 carbon atoms in the alkyl group. Particularly preferred ampholytic surfactants are N-cocoalkylaminopropionate, cocoacylaminoethylaminopropionate and C₁₂-C₁₈-acylsarcosine.

Nonionic surfactants comprise, as hydrophilic group, e.g. a polyol group, a polyalkylene glycol ether group or a combination of polyol and polyglycol ether group. Such compounds are, for example,
- addition products of from 2 to 50 mol of ethylene oxide and/or 0 to 5 mol of propylene oxide onto linear and branched fatty alcohols having 8 to 30 carbon atoms, onto fatty acids having 8 to 30 carbon atoms and onto alkylphenols having 8 to 15 carbon atoms in the alkyl group,
- addition products, terminally capped with a methyl or C₂-C₆-alkyl radical, of from 2 to 50 mol of ethylene oxide and/or 0 to 5 mol of propylene oxide onto linear and branched fatty alcohols having 8 to 30 carbon atoms, onto fatty acids having 8 to 30 carbon atoms and onto alkylphenols having 8 to 15 carbon atoms in the alkyl group, such as, for example, the grades available under the brand names Dehydol^{®} LS, Dehydol^{®} LT (Cognis),
- C₁₂-C₃₀-fatty acid mono- and diesters of addition products of from 1 to 30 mol of ethylene oxide onto glycerol,
- addition products of from 5 to 60 mol of ethylene oxide onto castor oil and hydrogenated castor oil, for example castor oil - hydrogenated+40 EO, as is commercially available, for example, under the trade name Cremophor CO 455 from SHC,
- polyol fatty acid esters, such as, for example, the commercial product Hydagen^{®} HSP (Cognis) or Sovermol grades (Cognis),
- alkoxylated triglycerides,
- alkoxylated fatty acid alkyl esters of the formula (V)

   R¹⁴CO-(OCH₂CHR¹⁵)_{w}OR¹⁶ (V)

   in which R¹⁴CO is a linear or branched, saturated and/or unsaturated acyl radical having 6 to 22 carbon atoms, R¹⁵ is hydrogen or methyl, R¹⁶ is linear or branched alkyl radicals having 1 to 4 carbon atoms and w is numbers from 1 to 20,
- amine oxides,
- hydroxy mixed ethers, as are described, for example, in DE-A 19738866,
- sorbitan fatty acid esters and addition products of ethylene oxide onto sorbitan fatty acid esters, such as, for example, the polysorbates,
- sugar fatty acid esters and addition products of ethylene oxide onto sugar fatty acid esters,
- addition products of ethylene oxide onto fatty acid alkanolamides and fatty amines,
- fatty acid N-alkylglucamides,
- alkyl polyglycosides corresponding to the general formula RO-(Z)ₓ, where R is alkyl, Z is sugar, and x is the number of sugar units. The alkyl polyglycosides which can be used according to the invention can contain just one specific alkyl radical R. Usually, however, these compounds are prepared starting from natural fats and oils or mineral oils. In this case, the alkyl radicals R present are mixtures corresponding to the starting compounds or corresponding to the particular work-up of these compounds.

Particular preference is given to those alkyl polyglycosides in which R consists
- essentially of C₈- and C₁₀-alkyl groups,
- essentially of C₁₂- and C₁₄-alkyl groups,
- essentially of C₈- to C₁₆-alkyl groups, or
- essentially of C₁₂- to C₁₆-alkyl groups, or
- essentially of C₁₆- to C₁₈-alkyl groups.

Sugar building blocks Z which can be used are any monosaccharides or oligosaccharides. Usually, sugars with 5 or 6 carbon atoms, and the corresponding oligosaccharides are used. Such sugars are, for example, glucose, fructose, galactose, arabinose, ribose, silose, lyxose, allose, altrose, mannose, gulose, idose, talose and sucrose. Preferred sugar building blocks are glucose, fructose, galactose, arabinose and sucrose; glucose is particularly preferred.

The alkyl polyglycosides which can be used according to the invention comprise on average 1.1 to 5 sugar units. Alkyl polyglycosides with x values of from 1.1 to 2.0 are preferred. Very particular preference is given to alkyl glycosides in which x is 1.1 to 1.8.

The alkoxylated homologs of the specified alkyl polyglycosides can also be used according to the invention. These homologs can comprise, on average, up to 10 ethylene oxide and/or propylene oxide units per alkyl glycoside unit.

Preferred nonionic surfactants have proven to be the alkylene oxide addition products onto saturated linear fatty alcohols and fatty acids having in each case 2 to 30 mol of ethylene oxide per mole of fatty alcohol or fatty acid. Preparations with excellent properties are likewise obtained if they comprise fatty acid esters of ethoxylated glycerol as nonionic surfactants.

These compounds are characterized by the following parameters. The alkyl radical R contains 6 to 22 carbon atoms and may either be linear or branched. Preference is given to primary linear and 2-methyl-branched aliphatic radicals.

Such alkyl radicals are, for example, 1-octyl, 1-decyl, 1-lauryl, 1-myristyl, 1-cetyl and 1-stearyl. Particular preference is given to 1-octyl, 1-decyl, 1-lauryl, 1-myristyl. When using so-called "oxo alcohols" as starting materials, compounds with an uneven number of carbon atoms in the alkyl chain predominate.

The compounds with alkyl groups used as surfactant may each be single substances. However, it is generally preferred, when producing these substances, to start from native vegetable or animal raw materials, thus giving mixtures of substances with different alkyl chain lengths that are dependent on the respective raw material.

In the case of the surfactants which constitute addition products of ethylene oxide and/or propylene oxide onto fatty alcohols or derivatives of these addition products, it is possible to use either products with a "normal" homolog distribution or those with a narrowed homolog distribution. "Normal" homolog distribution is understood here as meaning mixtures of homologs which are obtained in the reaction of fatty alcohol and alkylene oxide using alkali metals, alkali metal hydroxides or alkali metal alkoxides as catalysts. Narrowed homolog distributions, on the other hand, are obtained if, for example, hydrotalcites, alkaline earth metal salts of ethercarboxylic acids, alkaline earth metal oxides, hydroxides or alkoxides are used as catalysts. The use of products with a narrowed homolog distribution may be preferred.

According to a further embodiment of the invention, the surfactant-containing cleansing compositions can further comprise cationic surfactants of the quaternary ammonium type, the ester quat type and the amidoamine type.

Preferred quaternary ammonium compounds are ammonium halides, in particular chlorides and bromides, such as alkyltrimethylammonium chlorides, dialkyldimethyl-ammonium chlorides and trialkylmethylammonium chlorides, e.g. cetyltrimethylammonium chloride, stearyltrimethylammonium chloride, distearyldimethylammonium chloride, lauryl-dimethylammonium chloride, lauryldimethylbenzylammonium chloride and tricetylmethyl-ammonium chloride, and the imidazolium compounds known under the INCI names Quaternium-27 and Quaternium-83. The long alkyl chains of the abovementioned surfactants preferably have 10 to 18 carbon atoms.

Ester quats are known substances which contain both at least one ester function and also at least one quaternary ammonium group as structural element. Preferred ester quats are quaternized ester salts of fatty acids with triethanolamine, quaternized ester salts of fatty acids with diethanolalkylamines and quaternized ester salts of fatty acids with 1,2-dihydroxypropyldialkylamines. Such products are sold, for example, under the trade names Stepantex^{®}, Dehyquart^{®} and Armocare^{®}. The products Armocare^{®} VGH-70, an N,N-bis(2-palmitoyloxyethyl)dimethylammonium chloride, and Dehyquart^{®} F-75, Dehyquart^{®} C-4046, Dehyquart^{®} L80 and Dehyquart^{®} AU-35 are examples of such ester quats.

The alkylamidoamines are usually prepared by amidation of natural or synthetic fatty acids and fatty acid cuts with dialkylaminoamines. One compound from this group of substances which is particularly suitable according to the invention is the stearamidopropyldimethylamine commercially available under the name Tegoamid^{®} S 18.

The cationic surfactants are present in the compositions according to the invention preferably in amounts of from 0.05 to 10% by weight, based on the total composition. Amounts of from 0.1 to 5% by weight are particularly preferred.

In a particularly preferred embodiment of the invention, the surface-active cleansing compositions comprise a mixture of at least one anionic surfactant and at least one zwitterionic or amphoteric surfactant. Particular preference is given to a mixture of alkyl sulfates, alkyl ether sulfates and betaines.

Included in some exemplary cleansing compositions are one or more cleansing agents and one or more antimicrobial agents, which include substances that kill or inhibit the growth of microbes such as bacteria, fungi, viruses, and parasites. According to a preferred embodiment, the compositions include a surfactant as a cleansing agent, and triclosan (2,4,4'-trichloro-2'-hydroxydiphenyl ether) as an antimicrobial agent. Further preferred antimicrobial, antibacterial or germ-inhibiting substances are, in particular, organohalogen compounds as well as -halides, quaternary ammonium compounds and zinc compounds. Preferred halogenated phenol derivatives are e.g. hexachlorophene or 3,4,4'-trichlorocarbonilide, chlorhexidine (1,1'-hexamethylene-bis-[5-(4-chlorophenyl)]-biguanide), chlorhexidine gluconate, chlorhexidine digluconate, benzalkonium halides and cetylpyridinium chloride. Sodium phenolsulfonate, zinc phenolsulfonate, farnesol and benzyl alcohol are further preferred antimicrobial agents. Long-chain diols, especially 1,2-alkane-(C₈-C₁₈)-diols, glycerol mono-(C₆-C₁₆)-alkyl ethers or glycerol mono(C₈-C₁₈)-fatty acid esters, which have a very good skin and mucous membrane tolerability and are active against Corynebacteria, are also very preferred. Highly preferred is the glycerol 1-monoethylhexyl ether (INCI: ethylhexylglycerin), available under the trade name Sensiva SC 50. Numerous other cleansing agents and antimicrobial agents are known and may be included in the compositions of the present invention.

Additional components in the cleaning composition may be added to further impart a desired texture, to adjust the composition density, to provide a fragrance, for chelation or suspension, to increase the composition's usable life, to add color, to modify the pH, and so forth. For example, in an exemplary embodiment the cleansing composition includes a suspending agent such as an acrylates copolymer, a film forming agent such as a polyquaternium compound, or a humectant such as glycerin.

As previously discussed, the first plurality of beads that are included in the cleansing composition impart perceived tactility to a user applying the cleansing composition with a hand rubbing force, and the tactility is perceivable by the user for a limited predetermined duration that corresponds to a minimum adequate cleansing time period. Throughout the specification, the terms "adequate washing" and "adequate cleansing" refer to a washing or cleansing skin to the point at which transient contaminants are thoroughly removed from the skin surface. The limited predetermined duration is typically between about 10 and about 30 seconds. However, to ensure adequate cleaning, the limited predetermined duration is between at least 15 seconds, and is preferably at least 15 to 20 seconds, as recommended by the Centers for Disease Control.

The beads included in the first plurality of beads are formed as a uniform composition. During a washing event, the hand rubbing force exerted by the user causes the beads to wear down and disintegrate. The beads are made from a structural material that is hydrated by water content in the cleansing composition. Although hydration of the structural material is an important consideration when tailoring the beads to have a predetermined tactility-imparting life, the beads require the hand washing mechanical force in order to break down within the adequate cleaning time. More particularly, the mechanical force provided by the user causes the beads to disintegrate until they no longer impart perceivable tactility to the user during the range of seconds corresponding to the adequate cleaning time.

To provide perceived tactility to a user until adequate cleansing is completed, the beads included in the first plurality of beads have an average diameter ranging between about 400 and about 1200 µm. Smaller beads will provide inadequate tactility to a user. Beads having larger average diameters will provide tactility to a user, but the tactility may be uncomfortable or otherwise undesirable for users. It is also desirable to have a large number of beads, and smaller beads enable incorporation of a high bead concentration. According to a preferred embodiment, the beads have an average diameter ranging between about 500 and about 900 µm.

The beads included in the first plurality of beads are at least in part formed from a structural material that imparts the perceivable tactility. Although numerous materials exist as structural materials, preferred beads include a polymeric carbohydrate or derivative thereof, where cellulose and/or ethers of cellulose or alkyl cellulose are preferred structural materials. The structural material is preferably combined with at least one binder. Preferred binders are mono- and disaccharides as well as sugar alcohols, with mannitol being one preferred sugar alcohol. It is within the purview of the present invention that any material may be used as a structural material as long as it is sufficiently hard to provide a perceivable tactility to a user, and that it is sufficiently frangible to disintegrate when subjected to normal hand washing force to the extent that the tactility is no longer perceived after adequate washing has taken place.

According to another preferred embodiment, the tactile beads also impart, to a user applying the cleansing composition with hand rubbing force, a perceivable change in color to the cleansing composition. More particularly, the beads included in the first plurality of beads include a colorant that is released into the cleansing composition when subjected to the hand rubbing force. The color that the beads produce as they disintegrate during washing is not as important as the perceptibility of the cleansing composition color change. As previously mentioned, the tactile beads are formed as a uniform composition that disintegrates due to the hand rubbing force. Consequently, the color is introduced into the cleansing lather almost immediately after washing begins. Color is continuously released from the beads as they continue to disintegrate. Since the beads are adapted to be substantially completely disintegrated upon completion of the predetermined adequate washing period, in addition to no longer imparting perceivable tactility, the beads also no longer release additional color or impart additional color change once adequate washing has occurred.

Table 1 below provides preferred bead formulations for tactile beads that also include colorant, according to the embodiment in which the beads provide both perceivable tactility and color change. Of course, colorant may be omitted from the beads if it is desirable for the beads to only impart perceivable tactility.

**TABLE 1**

| Ingredient | BEAD A1 % (by weight) | BEAD A2 % (by weight) |
|---|---|---|
| Mannitol | 25-50 | > 50 |
| Cellulose | 25-50 | 25-50 |
| Hydroxypropyl Methylcellulose | < 1 | < 0.1 |
| Ultramarine | 25-50 | 0 |
| Chromium Hydroxide Green | 0 | 10-15 |
| Magnesium Stearate | 5-10 | 5-10 |

Referring now to FIG. 1, a graph depicts sensorial intensity provided to a user applying a hand rubbing force to beads included in a cleansing composition during a washing event, and correlates the sensorial intensity with the duration of the washing event. The trace labeled "TACTILITY" represents the perceived sensorial intensity for the tactility imparted by the beads in the first set of beads. As illustrated in the graph, the tactility is perceived with a fairly constant intensity for most of the washing time, and then drops sharply as the beads reach complete disintegration at a point that corresponds to an adequate washing period. If colorant is included in the tactile beads, the same trace corresponds to the perceived release of color since the beads are a substantially uniform composition. The colorant is released with at a fairly constant rate for most of the washing time, and the cleansing composition grows increasingly more colorful. Then, the release rate of the colorant drops sharply as the beads reach complete disintegration at a point that corresponds to an adequate washing period. As a user notices that the cleansing composition is no longer darkening or becoming more colorful, the user also recognizes the lack of tactility imparted by the beads. Both the tactile and color indicia signal to the user that adequate cleansing has occurred.

According to another preferred embodiment of the invention, two types of beads are incorporated into the cleansing composition. Beads of the first type are the tactility-imparting beads that may or may not include a colorant as discussed previously. The second plurality of beads are also entrained in the carrier and further impart, to the user applying the cleansing composition with hand rubbing force, perceivable change in color for the cleansing composition. More particularly, the beads included in the second plurality of beads include a colorant that is released into the cleansing composition when subjected to the hand rubbing force to impart the perceivable change in color. However, the change in color is perceived by the user in a different manner than that of the tactility-imparting beads that include a colorant. This is because instead of having a uniform composition, the beads included in the second plurality of beads preferably include a coating that delays release of the colorant into the cleansing composition. Furthermore, the beads included in the second plurality of beads are adapted to release most of the colorant into the cleansing composition prior to the minimum adequate cleansing time period. Hereinafter, the beads included in the second plurality of beads will be referred to as "color beads."

Returning to FIG. 1, the graph also includes a trace labeled "COLORANT RELEASE" that corresponds to the rate at which colorant is released from the color beads into the cleansing composition, and therefore the change in color perceived by the user. The coating on the color beads delays release of colorant until washing has taken place for several seconds. When the coating breaks due to the hand rubbing force, the colorant initially produces perceivable streaks of color as the hands move back and forth, and the color then mixes into the cleansing composition. The color beads have similar coating thicknesses, and consequently release colorant to produce the perceivable streaks at about the same time, which explains the sharp rise and fall of the corresponding trace in FIG. 1. The color beads are adapted to be substantially completely broken, and to therefore release all the colorant contained therein, when adequate cleansing has occurred. Complete bead breakage by the all of the beads before rinsing prevents formation of a visibly messy bead debris or residue in a sink when the cleansing composition is rinsed from the user's body. According to a preferred embodiment, the beads included in the second plurality of beads have more colorant than the first plurality of beads.

In another preferred embodiment, the first and second limited predetermined durations are between 10 and 30 seconds, preferably at least 15 to 20 seconds.

The color beads are preferably made from a structural material that is hydrated by water content in the cleansing composition. As with the tactility-imparting beads, hydration of the structural material in the color beads is an important consideration when tailoring the beads to have a predetermined color-imparting life. The color beads require the hand washing mechanical force in order for the coating to break down within the adequate cleaning time. More particularly, the mechanical force provided by the user causes the color beads to break and release color until they no longer impart perceivable color streaks to the user during the range of seconds corresponding to the adequate cleaning time.

To provide perceived color release to a user until adequate cleansing is completed, the color beads in the second plurality of beads have an average diameter ranging between 400 and 1200 µm. According to a preferred embodiment, the beads have an average diameter ranging between 425 and 1180 µm.

The color bead interior as well as the interior of the beads of the first plurality is at least in part formed from a structural material that is combined with colorant. Although numerous materials exist as structural materials, some beads may include a polysaccharide and a binder as exemplary structural materials. Some preferred polysaccharides are cellulose and ethers of cellulose or alkyl cellulose. Especially preferred ethers of cellulose or alkyl cellulose are selected from hydroxyethylcellulose, hydroxypropylcellulose, hydroxypropylmethylcellulose, methylcellulose, hydroxymethylcellulose and carboxymethylcellulose, as well as mixtures thereof. Preferred structural materials are characterized in that they comprise a combination of cellulose and at least one ether of cellulose or alkyl cellulose. Very preferred structural materials are characterized in that they comprise a combination of cellulose and at least one ether of cellulose or alkyl cellulose. Surprisingly, it has bee found that these structural material combinations are especially advantageous with regard to their disintegration time and behavior, which excellently fits to the need of the present invention. Especially preferred structural materials are characterized in that they comprise a combination of cellulose and at least one ether of cellulose or alkyl cellulose in a weight ratio of cellulose to ether(s) of cellulose and/or alkyl cellulose ranging from 1 to 1000, preferably from 5 to 500, more preferably from 10 to 250, especially preferred from 25 to 100 and most preferred from 50 to 100.

Preferred binder substances are selected from polyols, preferably from saccharose, lactose, erythritol, threitol, arabinitol, ribitol, xylitol, allitol, altitol, galactitol, iditol, isomalt, lactitol, mannitol and maltitol, each in its D-form as well in its L-form or as racemic mixture. Mixtures of the aforementioned substances can be preferred according to this invention. Very preferred are saccharose, lactose, mannitol, isomalt, maltitol, xylitol, lactitol and/or ribitol. Especially preferred binder substances are lactose and mannitol. It is within the purview of the present invention that numerous materials may be used as a structural material as long as it is sufficiently frangible to disintegrate when subjected to normal hand washing force.

Preferably, the color bead coating is at least in part formed from a polymer or copolymer. Some useful coating materials that may form the color bead exterior include Suitable coating materials are polymers or polymer mixtures from the group of the water-soluble polymers. Preferred substances from this group are selected from
a) the water-soluble nonionic polymers from the group of
   a1) polyvinylpyrrolidones,
   a2) vinylpyrrolidone-vinyl ester copolymers,
b) water-soluble amphoteric polymers from the group of
   b1) alkylacrylamide-acrylic acid copolymers
   b2) alkylacrylamide-methacrylic acid copolymers
   b3) alkylacrylamide-methylmethacrylic acid copolymers
   b4) alkylacrylamide-acrylic acid-alkylaminoalkyl-(meth)acrylic acid copolymers
   b5) alkylacrylamide-methacrylic acid-alkylamino-alkyl(meth)acrylic acid copolymers
   b6) alkylacrylamide-methylmethacrylic acid-alkyl-aminoalkyl(meth)acrylic acid copolymers
   b7) alkylacrylamide-alkyl methacrylate-alkylaminoethyl methacrylate-alkyl methacrylate copolymers
   b8) copolymers of
      b8i) unsaturated carboxylic acids
      b8ii) cationically derivatized unsaturated carboxylic acids
      b8iii) if desired, further ionic or nonionogenic monomers
c) water-soluble zwitterionic polymers from the group of
   c1) acrylamidoalkyltrialkylammonium chloride-acrylic acid copolymers and their alkali metal and ammonium salts
   c2) acrylamidoalkyltrialkylammonium chloride-methacrylic acid copolymers and their alkali metal and ammonium salts
   c3) methacroylethyl betaine-methacrylate copolymers
d) water-soluble anionic polymers from the group of
   d1) vinyl acetate-crotonic acid copolymers
   d2) vinylpyrrolidone-vinyl acrylate copolymers
   d3) acrylic acid-ethyl acrylate-N-tert-butylacrylamide terpolymers
   d4) graft polymers of vinyl esters, esters of acrylic acid or methacrylic acid alone or in a mixture, copolymerized with crotonic acid, acrylic acid or methacrylic acid with polyalkylene oxides and/or polyalkylene glycols
   d5) grafted and crosslinked copolymers from the copolymerization of
      d5i) at least one monomer of the nonionic type,
      d5ii) at least one monomer of the ionic type,
      d5iii) polyethylene glycol, and
      d5iv) a crosslinker
   d6) copolymers obtained by copolymerizing at least one monomer from each of the three following groups:
      d6i) esters of unsaturated alcohols and short-chain saturated carboxylic acids and/or esters of short-chain saturated alcohols and unsaturated carboxylic acids,
      d6ii) unsaturated carboxylic acids,
      d6iii) esters of long-chain carboxylic acids and unsaturated alcohols and/or esters of the carboxylic acids of group d6ii) with saturated or unsaturated, straight-chain or branched C₈₋₁₈ alcohol
   d7) terpolymers of crotonic acid, vinyl acetate and an allyl or methallyl ester
   d8) tetra- and pentapolymers of
      d8i) crotonic acid or allyloxyacetic acid
      d8ii) vinyl acetate or vinyl propionate
      d8iii) branched allyl or methallyl esters
      d8iv) vinyl ethers, vinyl esters or straight-chain allyl or methallyl esters
   d9) crotonic acid copolymers with one or more monomers from the group consisting of ethylene, vinylbenzene, vinyl methyl ether, acrylamide, and water-soluble salts thereof
   d10) terpolymers of vinyl acetate, crotonic acid, and vinyl esters of a saturated aliphatic α-branched monocarboxylic acid
e) water-soluble cationic polymers from the group of
   e1) quaternized cellulose derivatives
   e2) polysiloxanes with quaternary groups
   e3) cationic guar derivatives
   e4) polymeric dimethyldiallylammonium salts and their copolymers with esters and amides of acrylic acid and methacrylic acid
   e5) copolymers of vinylpyrrolidone with quaternized derivatives of dialkylaminoacrylate and -methacrylate
   e6) vinylpyrrolidone-methoimidazolinium chloride copolymers
   e7) quaternized polyvinyl alcohol
   e8) polymers indicated under the INCI designations Polyquaternium 2, Polyquaternium 17, Polyquaternium 18, and Polyquaternium 27.
Water-soluble polymers in the context of the invention are those polymers which are soluble in water to an extent of more than 2.5% by weight at 20 °C.
The beads dispersed in the inventive cleansers are preferably coated with a polymer or polymer mixture, the polymer (and accordingly the overall mixture) or at least 50% by weight of the polymer mixture (and thus at least 50% of the coating) being selected from certain polymers. The coating consists entirely or to an extent of at least 50% of its weight of water-soluble polymers from the group of the nonionic, amphoteric, zwitterionic, anionic and/or cationic polymers. In a further preferred embodiment, the coating of the phosphate consists of a further inorganic salt which comprises one of the polymers mentioned as a binder. Preferred polymers from these groups have been listed above and are described in detail below.
Water-soluble polymers which are preferred in accordance with the invention are nonionic. Examples of suitable nonionogenic polymers are the following:
○ polyvinylpyrrolidones, as sold, for example, under the name Luviskol® (BASF). Polyvinylpyrrolidones are preferred nonionic polymers in the context of the invention.
Polyvinylpyrrolidones [poly(1-vinyl-2-pyrrolidinones)], abbreviated PVP, are polymers which are prepared by free-radical polymerization of 1-vinylpyrrolidone by processes of solution or suspension polymerization using free-radical initiators (peroxides, azo compounds). The ionic polymerization of the monomer affords only products having low molar masses. Commercial polyvinylpyrrolidones have molar masses in the range of approx. 2500-750,000 g/mol, which are characterized by specifying the K values and, depending on the K value, have glass transition temperatures of 130-175°. They are supplied as white, hygroscopic powders or as aqueous solutions. Polyvinylpyrrolidones are readily soluble in water and a multitude of organic solvents (alcohols, ketones, glacial acetic acid, chlorohydrocarbons, phenols, etc).
Vinylpyrrolidone-vinyl ester copolymers, as sold, for example, under the trademark Luviskol® (BASF). Luviskol® VA 64 and Luviskol® VA 73, each vinylpyrrolidone-vinyl acetate copolymers, are particularly preferred nonionic coating polymers.
Further coating polymers preferred in accordance with the invention are water-soluble amphopolymers. The generic term amphopolymers embraces amphoteric polymers, i.e. polymers which includes both free amino groups and free -COOH or SO₃H groups in the molecule and which are capable of forming internal salts; zwitterionic polymers whose molecule includes quaternary ammonium groups and -COO⁻ or -SO₃⁻ groups, and those polymers which contain -COOH or SO₃H groups and quaternary ammonium groups. An example of an amphopolymer which can be used in accordance with the invention is the acrylic resin obtainable under the name Amphomer®, which is a copolymer of tert-butylaminoethyl methacrylate, N-(1,1,3,3-tetra-methylbutyl)-acrylamide, and two or more monomers from the group consisting of acrylic acid, methacrylic acid and their simple esters. Likewise preferred amphopolymers are composed of unsaturated carboxylic acids (e.g. acrylic and methacrylic acid), cationically derivatized unsaturated carboxylic acids (e.g. acrylamidopropyltrimethylammonium chloride) and, if desired, further ionic or nonionogenic monomers, as can be taken, for example, from DE-A 39 29 973 and the prior art cited therein. Terpolymers of acrylic acid, methyl acrylate and methacrylamidopropyltrimonium chloride, as obtainable commercially under the name Merquat® 2001 N, are particularly preferred amphopolymers in accordance with the invention. Further suitable amphoteric polymers are, for example, the octylacrylamide-methyl methacrylate-tert-butylaminoethyl methacrylate-2-hydroxypropyl methacrylate copolymers available under the names Amphomer® and Amphomer® LV-71 (DELFT NATIONAL)
Suitable zwitterionic polymers are, for example, acrylamidopropyltrimethylammonium chloride-acrylic acid or -methacrylic acid copolymers and their alkali metal salts and ammonium salts. Further suitable zwitterionic polymers are methacryloylethyl betaine-methacrylate copolymers, which are obtainable commercially under the name Amersette® (AMERCHOL).
Anionic polymers suitable in accordance with the invention include:
○ Vinyl acetate-crotonic acid copolymers, as commercially available, for example, under the names Resyn® (NATIONAL STARCH), Luviset® (BASF) and Gafset® (GAF).
○ Vinylpyrrolidone-vinyl acrylate copolymers, obtainable for example under the trademark Luviflex® (BASF). A preferred polymer is the vinylpyrrolidone-acrylate terpolymer obtainable under the name Luviflex® VBM-35 (BASF).
○ Acrylic acid/ethyl acrylate/N-tert-butylacrylamide terpolymers, which are sold, for example, under the name Ultrahold® strong (BASF).
○ Graft polymers of vinyl esters, esters of acrylic acid or methacrylic -acid alone or in a mixture, copolymerized with crotonic acid, acrylic acid or methacrylic acid with polyalkylene oxides and/or polyalkylene glycols.
Such grafted polymers of vinyl esters, esters of acrylic acid or methacrylic acid alone or in a mixture with other copolymerizable compounds onto polyalkylene glycols are obtained by polymerization under hot conditions in homogeneous phase, by stirring the polyalkylene glycols into the monomers of the vinyl esters, esters of acrylic acid or methacrylic acid, in the presence of free-radical initiators.
Suitable vinyl esters have been found to be, for example, vinyl acetate, vinyl propionate, vinyl butyrate, vinyl benzoate, and suitable esters of acrylic acid or methacrylic acid have been found to be those which are obtainable with low molecular weight aliphatic alcohols, i.e., in particular, ethanol, propanol, isopropanol, 1-butanol, 2-butanol, 2-methyl-1-propanol, 2-methyl-2-propanol, 1-pentanol, 2-pentanol, 3-pentanol, 2,2-dimethyl-1-propanol, 3-methyl-1-butanol, 3-methyl-2-butanol, 2-methyl-2-butanol, 2-methyl-1-butanol, 1-hexanol.
Useful polyalkylene glycols include in particular polyethylene glycols and polypropylene glycols. Polyethylene glycols are polymers of ethylene glycol which satisfy the general formula V H-(O-OH₂-CH₂)ₙ(V) in which n may assume -OH values between 1 (ethylene glycol) and several thousand. For polyethylene glycols there exist various nomenclatures, which may lead to confusion. It is common practice in industry to specify the average relative molar weight after "PEG", so that "PEG 200" characterizes a polyethylene glycol having a relative molar mass of approx. 190 to, approx. 210. For cosmetic ingredients, a different nomenclature is used, in which the abbreviation PEG is followed by a hyphen which is followed directly by a number which corresponds to the number n in the abovementioned formula V. According to this nomenclature (known as the INCI nomenclature, CTFA International Cosmetic Ingredient Dictionary and Handbook, 5th Edition, The Cosmetic, Toiletry and Fragrance Association, Washington, 1997), for example, PEG-4, PEG-6, PEG-8, PEG-9, PEG-10, PEG-12, PEG-14 and PEG-16 may be used. Polyethylene glycols are available commercially, for example, under the trade names Carbowax® PEG 200 (Union Carbide), Emkapol® 200 (ICI Americas), Lipoxol® 200 MED (HUELS America), Polyglycol® E-200 (Dow Chemical); Alkapol® PEG 300 (Rhône-Poulenc), Lutrol® E300 (BASF), and the corresponding trade names with higher numbers.
In particular, it is possible to use the vinyl acetate copolymers grafted onto polyethylene glycols and the polymers of vinyl acetate and crotonic acid grafted onto polyethylene glycols.
○ Grafted and crosslinked copolymers from the copolymerization of
   ■ i) at least one monomer of the nonionic type,
   ■ ii) at least one monomer of the ionic type,
   ■ iii) polyethylene glycol, and
   ■ iv) a crosslinker
The polyethylene glycol used has a molecular weight of between 200 and several million, preferably between 300 and 30,000. The nonionic monomers may be of very different types and preference is given among them to the following: vinyl acetate, vinyl stearate, vinyl laurate, vinyl propionate, allyl stearate, allyl laurate, diethyl maleate, allyl acetate, methyl methacrylate, cetyl vinyl ether, stearyl vinyl ether, and 1-hexene.
The ionic monomers may equally be of very different types, and particular preference among these is given to the presence in the graft polymers of crotonic acid, allyloxyacetic acid, vinylacetic acid, maleic acid, acrylic acid, and methacrylic acid.
Preferred crosslinkers are ethylene glycol dimethacrylate, diallyl phthalate, ortho-, meta- and paradivinylbenzene, tetraallyloxyethane, and polyallylsaccharoses containing 2 to 5 allyl groups per molecule of saccharin.
The above-described grafted and crosslinked copolymers are formed preferably from:
○ i) from 5 to 85% by weight of at least one monomer of the nonionic type,
○ ii) from 3 to 80% by weight of at least one monomer of the ionic type,
○ iii) from 2 to 50% by weight, preferably from 5 to 30% by weight, of polyethylene. glycol, and
○ iv) from 0.1 to 8% by weight of a crosslinker, the percentage of the crosslinker depending on the ratio of the overall weights of i), ii) and iii).
○ Copolymers obtained by copolymerizing at least one monomer from each of the three following groups:
   ■ i) esters of unsaturated alcohols and short-chain saturated-carboxylic acids and/or esters of short-chain saturated alcohols and unsaturated carboxylic acids,
   ■ ii) unsaturated carboxylic acids,
   ■ iii) esters of long-chain carboxylic acids and unsaturated alcohols and/or esters of the carboxylic acids of group ii) with saturated or unsaturated, straight-chain or branched C₈₋₁₈ alcohol.
Short-chain carboxylic acids and alcohols in this context are those having from 1 to 8 carbon atoms, and the carbon chains of these compounds may optionally be interrupted by divalent heteroatomic groups such as -O-, NH-, and -S-.
○ Terpolymers of crotonic acid, vinyl acetate, and an allyl or methallyl ester
The aforementioned terpolymers result preferably from the copolymerization of from 7 to 12% by weight of crotonic acid, from 65 to 86% by weight, preferably from 71 to 83% by weight, of vinyl acetate and from 8 to 20% by weight, preferably from 10 to 17% by weight, of allyl or methallyl esters.
○ Tetra- and pentapolymers of
   ■ i) crotonic acid or allyloxyacetic acid
   ■ ii) vinyl acetate or vinyl propionate
   ■ iii) branched allyl or methallyl esters
   ■ iv) vinyl ethers, vinyl esters or straight-chain allyl or methallyl esters.
○ Crotonic acid copolymers with one or more monomers from the group of ethylene, vinylbenzene, vinyl methyl ether, acrylamide and the water-soluble salts thereof.
○ Terpolymers of vinyl acetate, crotonic acid and vinyl esters of a saturated aliphatic α-branched monocarboxylic acid.
Further polymers which can be used with preference as coating constituents are cationic polymers. Among the cationic polymers, preference is given to the permanently cationic polymers. "Permanently cationic" refers in accordance with the invention to those polymers which, irrespective of the pH of the composition (i.e. both of the coating and of the beads), have a cationic group. These are generally polymers which include a quaternary nitrogen atom, for example in the form of an ammonium group.
Preferred cationic polymers are, for example:
Cationic polymers which are preferred in accordance with the invention are quaternized cellulose derivatives and also polymeric dimethyldiallylammonium salts and copolymers thereof. Cationic cellulose derivatives, especially the commercial product Polymer® JR 400, are very particularly preferred cationic polymers.
○ quaternized cellulose derivatives, as available commercially under the names Celquat® and Polymer JR®. The compounds Celquat® H 100, Celquat® L 200 and Polymer JR® 400 are preferred quaternized cellulose derivatives,
○ polysiloxanes with quaternary groups, for example the commercially available products Q2-7224 (manufacturer: Dow Corning; a stabilized trimethylsilylamo-dimethicone), Dow Corning® 929 emulsion (comprising a hydroxyamino-modified silicone, also referred to as amodimethicone), SM-2059 (manufacturer: General Electric), SLM-55067 (manufacturer: Wacker), and Abil®-Quat 3270 and 3272 (manufacturer: Th. Goldschmidt; diquaternary polydimethylsiloxanes, Quaternium-80),
○ cationic guar derivatives, such as in particular the products sold under the trade names Cosmedia® Guar and Jaguar®,
○ polymeric dimethyldiallylammonium salts and their copolymers with esters and amides of acrylic acid and methacrylic acid. The products available commercially under the names Merquat® 100 (poly(dimethyldiallylammonium chloride)) and Merquat® 550 (dimethyldiallylammonium chloride-acrylamide copolymer) are examples of such cationic polymers.
○ Copolymers of vinylpyrrolidone with quaternized derivatives of dialkylamino acrylate and methacrylate, for example diethyl sulfate-quaternized vinylpyrrolidone-dimethylamino methacrylate copolymers. Such compounds are available commercially under the names Gafquat® 734 and Gafquat® 755.
○ Vinylpyrrolidone-methoimidazolinium chloride copolymers, as supplied under the name Luviquat®.
○ Quaternized polyvinyl alcohol and also polymers known under the names
○ Polyquaternium 2
○ Polyquaternium 17,
○ Polyquaternium 18, and
○ Polyquaternium 27, having quaternary nitrogen atoms in the polymer main chain. These polymers are designated in accordance with the INCI nomenclature; detailed information can be found in the CTFA International Cosmetic Ingredient Dictionary and Handbook, 5th Edition, The Cosmetic, Toiletry and Fragrance Association, Washington, 1997, which is expressly incorporated herein by reference.

A particularly preferred coating material in the context of the present application for the dispersed beads is polyvinyl alcohol (PVA). Another particularly preferred coating material in the context of the present application for the dispersed beads is a copolymer of methyl vinyl ether and maleic anhydride (PVM/MA copolymer).

Preferred cleansing compositions according to the present invention are characterized in that the cleansing composition has a total bead weight less than 5%, preferably less than 1.2%, with reference to the weight of the total cleansing composition. Also preferred is a total bead weight of 0.2 to 4.5 % by weight, more preferred is a total bead weight of 0.5 to 4.0 % by weight, further 1.0 to 3.5 % by weight, further 1.5 to 3.0 % by weight, further 2.0 to 2.5 % by weight, with reference to the weight of the total cleansing composition.

Table 2 below provides an exemplary bead formulation for color beads.

**TABLE 2**

| Ingredient | BEAD B 1 % (by weight) | BEAD B2 % (by weight) |
|---|---|---|
| Cellulose | 15-20 | 15-20 |
| Hydroxypropyl Methylcellulose | 4-9 | 4-9 |
| Lactose | 25-32 | 25-32 |
| Ultramarine | 45-55 | 0 |
| Chromium Hydroxide Green | 0 | 45-55 |
| PVA | 0.1-2.0 | 0 |
| PVM/MA Copolymer | 0 | 1-4 |

Examples

The following hand and body cleansers according to the invention were prepared. The indicated numbers refer to % by weight.

| | A | B | C | D | E |
|---|---|---|---|---|---|
| | % telquel | % telquel | % telquel | % telquel | % telquel |
| Texapon^{®} N 70¹ | 12 | 10 | 8 | 10 | 14 |
| Dehyton^{®} PS² | 9 | - | 4 | 10 | 2 |
| Rewoteric^{®} AMC³ | - | 7 | 6 | - | - |
| Plantacare^{®} 818⁴ | 2 | 1.5 | - | - | 5 |
| Conditioner^{®} P7⁵ | 1 | 0.5 | - | 2 | - |
| Panthenol | 0.2 | - | 0.1 | - | - |
| Euperlan^{®} PK 810⁶ | 0.5 | - | - | 2 | 2 |
| Acusol^{®} OP 301⁷ | 2 | - | 3 | 0.5 | - |
| Vitamin E | - | 0.1 | 0.2 | - | - |
| BEADS A1 | 3 | - | - | 1.5 | - |
| BEADS A2 | - | 1.5 | 1.5 | - | 2 |
| BEADS B1 | 1 | 1 | - | - | 2 |
| BEADS B2 | - | - | 0.8 | 0.9 | - |
| Triclosan | - | - | 0.5 | - | - |
| Sensiva SC 50 | 1.0 | - | - | - | - |
| Chlorhexidine digluconate | - | 1.0 | - | - | - |
| Ethanol | - | 10 | - | 15 | 10 |
| Water | ad 100 | ad 100 | ad 100 | ad 100 | ad 100 |

| | | | | | |
|---|---|---|---|---|---|
| ¹ sodium lauryl ether sulfate, 2 EO (INCI: SODIUM LAURETH SULFATE); AS: 68%; Cognis ² coconut fatty acid amidoethyl N-2-hydroxyethylglycine sodium salt (INCI: DISODIUM COCOAMPHODIACETATE); AS: 37.5%; Cognis ³ hydroxyethyl N-cocoalkylamidoethyl carboxymethylglycinate sodium salt (INCI: SODIUM COCOAMPHOACETATE); AS: 30.5-32%; Evonik Degussa ⁴ C₈-C₁₄-alkyl polyglucoside (INCI: COCO-GLUCOSIDE); AS: 51-53%; Cognis ⁵ acrylamide dimethyldiallylammonium chloride copolymer (INCI: POLYQUATERNIUM-7); 3V Sigma ⁶ pearlizing agent (INCI: AQUA; GLYCOL DISTEARATE; SODIUM LAURETH SULFATE; COCAMIDE MEA; LAURETH-10, FORMIC ACID); Cognis ⁷ styrene acrylate copolymer (INCI: STYRENE/ACRYLATES COPOLYMER); AS: 39-41%; Rohm & Haas | | | | | |

## Claims

1. A cleansing composition, comprising
a carrier; and
a first plurality of beads entrained in the carrier and imparting, to a user applying the cleansing composition with hand rubbing force, perceivable tactility for a limited predetermined duration that corresponds to a minimum adequate cleansing time period.

2. The cleansing composition according to claim 1, wherein the limited predetermined duration is between 10 and 30 seconds, preferably at least 15 to 20 seconds.

3. The cleansing composition according to claim 1 or claim 2, wherein the beads included in the first plurality of beads have an average diameter ranging between 400 and 1200 µm, preferably between 500 and 900 µm.

4. The cleansing composition according to any of claims 1 - 3, wherein the beads included in the first plurality of beads comprise a structural material that imparts the perceivable tactility, the structural material comprising at least one polymeric carbohydrate or a derivative thereof.

5. The cleansing composition according to any of claims 1 - 4, wherein the at least one polymeric carbohydrate or derivative thereof is selected from the group consisting of cellulose, and ethers of cellulose or alkyl cellulose.

6. The cleansing composition according to any of claims 1 - 5, wherein the structural material further is combined with at least one binder.

7. The cleansing composition according to any of claims 1 - 6, wherein the beads included in the first plurality of beads further impart, to a user applying the cleansing composition with hand rubbing force, perceivable change in color for the cleansing composition.

8. The cleansing composition according to any of claims 1 - 7, wherein the beads included in the first plurality of beads comprise a colorant that is released into the cleansing composition when subjected to the hand rubbing force to impart the perceivable change in color.

9. A cleansing composition according to any of claims 1 - 8, comprising
a second plurality of beads entrained in the carrier and imparting, to the user applying the cleansing composition with hand rubbing force, perceivable change in color for the cleansing composition for a second limited predetermined duration that is substantially equal to the duration that the tactility imparted by the first plurality of beads is perceivable to the user.

10. The cleansing composition according to claim 9, wherein the beads included in the second plurality of beads comprise a colorant that is released into the cleansing composition when subjected to the hand rubbing force to impart the perceivable change in color.

11. The cleansing composition according to claim 9 or claim 10, wherein the beads included in the second plurality of beads include a coating that delays release of the colorant into the cleansing composition.

12. The cleansing composition according to any of claims 9 - 11, wherein the beads included in the second plurality of beads have more colorant than the first plurality of beads.

13. The cleansing composition according to any of claims 9 - 12, wherein the first and second limited predetermined durations are between 10 and 30 seconds, preferably at least 15 to 20 seconds.

14. The cleansing composition according to any of claims 9 - 13, wherein the beads included in the second plurality of beads have an average diameter ranging between 400 and 1200 µm, preferably between 425 and 1180 µm.

15. The cleansing composition according to any of claims 9 - 14, wherein the beads included in the first and second plurality of beads comprise a structural material, the structural material comprising a polymeric carbohydrate or derivative thereof.

16. The cleansing composition according to any of claims 9 - 15, wherein the polymeric carbohydrate or derivative thereof included in the structural material for the beads in the second plurality of beads includes at least one compound selected from the group consisting of cellulose, and ethers of cellulose or alkyl cellulose.

17. The cleansing composition according to any of claims 1 - 16, wherein the binder included in the first and/or second plurality of beads is a polyol.

18. The cleansing composition according to claim 17, wherein the binder polyol is selected from the group, consisting of saccharose, lactose, erythritol, threitol, arabinitol, ribitol, xylitol, allitol, altitol, galactitol, iditol, isomalt, lactitol, mannitol and maltitol, each in its D-form as well in its L-form or as racemic mixture.

19. The cleansing composition according to claim any of claims 1 - 18, wherein the cleansing composition has a total bead weight less than 5%, preferably less than 1.2%.
